(19) Europäisches Patentamt
European Patent Office
Office européen des brevets

(11) Veröffentlichungsnummer: **0 464 488 B1**

(12) **EUROPÄISCHE PATENTSCHRIFT**

(45) Veröffentlichungstag der Patentschrift: **27.09.95**

(21) Anmeldenummer: **91110142.6**

(22) Anmeldetag: **20.06.91**

(51) Int. Cl.[6]: **C07C 233/49**, C07D 277/12, C08F 20/56, C08F 114/18, C07B 57/00, C08F 20/54

Die Akte enthält technische Angaben, die nach dem Eingang der Anmeldung eingereicht wurden und die nicht in dieser Patentschrift enthalten sind.

(54) **Optisch aktive N-aplha-Fluoracryloylaminosäure-Derivate, ihre Herstellung, die daraus hergestellten optisch aktiven Polymeren und deren Verwendung zur Spaltung von Racematen.**

(30) Priorität: **03.07.90 DE 4021108**

(43) Veröffentlichungstag der Anmeldung:
**08.01.92 Patentblatt 92/02**

(45) Bekanntmachung des Hinweises auf die Patenterteilung:
**27.09.95 Patentblatt 95/39**

(84) Benannte Vertragsstaaten:
**AT BE CH DE DK ES FR GB GR IT LI LU NL SE**

(56) Entgegenhaltungen:
**EP-A- 0 282 770**
**EP-A- 0 282 770**
**EP-A- 0 379 917**
**DE-A- 1 570 802**

**CHROMATOGRAPHIC SCIENCE SERIES Bd. 40, 1988, NEW YORK ; BASEL Seiten 179 - 198 BLASCHKE 'Substituted Polyacrylamides as Chiral Phases for the Resolution of Drugs'**

(73) Patentinhaber: **BAYER AG**

**D-51368 Leverkusen (DE)**

(72) Erfinder: **Grosser, Rolf, Dr.**
**Gellertstrasse 9**
**W-5090 Leverkusen (DE)**
Erfinder: **Lange, Walter, Dr.**
**Auerstrasse 7**
**W-5000 Köln 60 (DE)**
Erfinder: **Bömer, Bruno, Dr.**
**Max-Planck-Strasse 53**
**W-5060 Bergisch Gladbach (DE)**
Erfinder: **Arlt, Dieter, Prof. Dr.**
**Rybniker Strasse 2**
**W-5000 Köln 80 (DE)**
Erfinder: **Bielefeldt, Dietmar, Dr.**
**Beuthener Strasse 13**
**W-4030 Ratingen 6 (DE)**

**EP 0 464 488 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue optisch aktive N-α-Fluoracryloyl-aminosäure-Derivate, Verfahren zu ihrer Herstellung, ihre Polymerisation zu optisch aktiven Polymeren und die Verwendung dieser optisch aktiven Polymere als Adsorbentien für die chromatographische Trennung von Racematen in ihre Enantiomere.

In den letzten Jahren gewinnt die Trennung von Wirkstoff-Racematen zunehmend an Bedeutung, weil sich gezeigt hat, daß die Enantiomere eines Wirkstoff-Racemats sich häufig in ihren biologischen Wirkungen und Nebenwirkungen unterscheiden.

Neben den klassischen Verfahren zur Racematspaltung hat sich in letzter Zeit die chromatographische Racemattrennung besonders bewährt. Neben Naturstoff-Derivaten z.B. auf Cellulose-Basis sind vermehrt synthetische optisch aktive polymere (Meth)acrylamide als Adsorbentien eingesetzt worden (Übersicht: G. Blaschke, Chromatogr. Sci. 1988, 40, 179-198).

In DE-A-1 570 802 werden schon Acrylsäurederivate beschrieben, die in der breiten Definition der allgemeinen Formel mit einigen der erfindungsgemäßen ausgewählten Verbindungen überlappen. Fluoracrylsäurederivate sind jedoch dort expressis verbis nicht genannt.

Bei der Anwendung der bekannten Methoden hat sich jedoch gezeigt, daß diese entweder nur eine unzureichende Wirkung aufweisen oder nur eine auf bestimmte Racemate begrenzte Wirksamkeit aufweisen.

Es wurde nun überraschend gefunden, daß Polymere aus optisch aktiven α-Fluoracrylsäureamiden von Aminosäureestern bzw. Aminosäureamiden Adsorbentien mit sehr guten Racemattrenneigenschaften darstellen.

Überraschenderweise führt der Ersatz des Wasserstoffatoms bzw. der Methylgruppe in den (Meth)-acrylamiden durch das stark elektronegative Fluoratom nicht zu einem Verlust an Trennwirkung, wie man aufgrund des gegensätzlichen elektronischen Effekts und der Nähe des Fluoratoms zur Amidgruppe, die ja über Wasserstoffbrücken mit den Enantiomeren des zu trennenden Racemats wechselwirken muß, annehmen würde.

Man beobachtet vielmehr, daß solche neuen polymeren Fluoracrylamide in vielen Fällen Racemate in die Enantiomere trennen können, die das entsprechende polymere (Meth)acrylamid-Adsorbens ungetrennt passieren.

Somit kommt man in unerwarteter Weise durch die erfindungsgemäßen polymeren Fluoracrylsäure-Derivate zu Adsorbentien für die chromatographische Racemattrennung, die sich in ihren Eigenschaften von den entsprechenden (Meth)acrylamiden deutlich unterscheiden.

Die Erfindung betrifft optisch aktive N-α-Fluoracryloylaminosäurederivate der Formel (I)

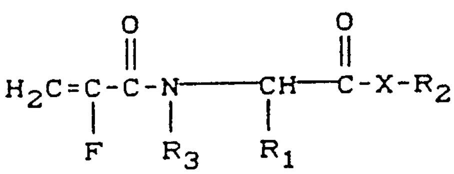

(I),

in der

- $R_1$ für einen geradkettigen oder verzweigten $C_1$-$C_8$-Alkyl-, einen $C_7$-$C_{12}$-Aralkyl-, einen $C_3$-$C_{10}$-Cycloalkyl-, einen $C_6$-$C_{14}$-Aryl- oder einen Furyl-, Thienyl- oder Pyridylrest steht, die gegebenenfalls durch Benzyloxycarbonyl, Alkoxycarbonyl mit bis zu 6 C-Atomen, Hydroxyl, Alkyl, Cycloalkyl oder Alkoxy mit jeweils bis zu 6 C-Atomen, Halogen, Phenoxy, Benzoxy, Acylamino mit bis zu 8 C-Atomen oder durch Carbonylalkoxy mit bis zu 6 C-Atomen substituiert sind,
- $R_3$ für Wasserstoff oder $C_1$-$C_4$-Alkyl steht oder zusammen mit $R_1$ eine Tri- oder Tetramethylengruppe bildet,
- X Sauerstoff oder eine $NR_4$-Gruppe bedeutet, in der
- $R_4$ für Wasserstoff oder geradkettiges oder verzweigtes $C_1$-$C_4$-Alkyl steht oder zusammen mit $R_2$ und dem Stickstoffatom einem 5- bis 7-gliedrigen Ring bildet, der gegebenenfalls durch eine Alkoxycarbonylgruppe mit bis zu 6 Kohlenstoffatomen oder durch ein oder zwei Alkylgruppen mit je 1 bis 4 Kohlenstoffatomen substituiert ist,
- $R_2$ einen geradkettigen oder verzweigten $C_1$-$C_{22}$-Alkyl-, $C_7$-$C_{12}$-Aralkyl-, $C_3$-$C_{10}$-Cycloalkyl-, $C_6$-$C_{14}$-Aryl- oder einen Terpenylrest darstellt, die gegebenenfalls substituiert sind durch Halogen, Alkyl oder Alkoxy mit jeweils 1 bis 4 C-Atomen.

Als Reste $R_1$ seien beispielhaft genannt für gegebenenfalls substituiertes geradkettiges oder verzweigtes $C_1$-$C_8$-Alkyl der Methyl, Ethyl, i-Propyl, n-Propyl, n-Butyl, i-Butyl, t-Butyl, 1-Hydroxymethyl, 1-Hydroxyethyl, 3-Acetylaminopropyl-, 4-Benzoyl-aminobutyl, t-Butoxymethyl, Benzyloxymethyl-, 2-Methoxycarbonylmethyl-, 3-Methoxycarbonylethyl-, 4-t-Butoxycarbonylpropyl-Rest oder beispielsweise der 2-Cyclohexylethyl-Rest; für gegebenenfalls substituiertes $C_7$-$C_{12}$-Aralkyl der Benzyl-, 1-Naphthylmethyl-, 2-Naphthylmethyl-, 3-Indolylmethyl-, 4-Hydroxybenzyl-, 2-Phenylbutyl-, 3-Phenylbutyl- und der 4-Phenylbutyl-Rest; für gegebenenfalls substituiertes geradkettiges oder verzweigtes $C_4$-$C_{10}$-Cycloalkyl der Cyclopropyl-, Cyclobutyl-, Cyclopentyl-, Cyclohexyl-oder Decahydronaphthyl-Rest; für einen gegebenenfalls substituierten $C_6$-$C_{14}$-Aryl-Rest der Phenyl-, der 1-Naphthyl- und der 2-Naphthyl-Rest.

Als Reste $R_4$ seien beispielhaft der Wasserstoff-, Methyl-, Ethyl-, n-Propyl-, i-Propyl-, n-Butyl- und der i-Butyl-Rest genannt, während als gegebenenfalls substituierter Ring exemplarisch der Pyrrolidinyl-, Piperidinyl-, Morpholinyl-, 2-Alkoxycarbonylpyrrolidinyl- und der 2-Alkoxymethylpyrrolidinyl-Rest angeführt seien, mit jeweils 1 bis 4 C-Atomen in der Alkoxygruppe.

Beispiele für Reste $R_2$ sind gegebenenfalls halogen- oder alkoxy-($C_1$-$C_4$)-substituierte geradkettige oder verzweigte $C_1$-$C_{22}$-Alkylreste wie der Methyl-, Ethyl-, Trifluorethyl-,2-Chlorethyl-, n-Propyl-, i-Propyl-, Hexafluorisopropyl-, 2-Methoxy-ethyl-, 2-t-Butoxy-ethyl-, n-Butyl-, i-Butyl-, t-Butyl-, 2-Octyl-, 2-Nonyl-Rest, Stearyl- und der Behenyl-Rest; gegebenenfalls substituierte $C_7$-$C_{12}$-Aralkyl-Reste wie der Benzyl-, 1-Phenylethyl- und der 1-Naphthylethyl-Rest; gegebenenfalls halogen-, alkoxy-($C_1$-$C_4$)- oder alkyl-($C_1$-$C_4$)- substituierte $C_4$-$C_{10}$-Cycloalkyl-Reste wie der Cyclobutyl-, Cyclopentyl-, Cyclohexyl-, 2,6-Dimethylcyclohexyl-, 3,5-Dimethylcyclohexyl-,4-t-Butylcyclohexyl- oder der Decahydronaphthyl-Rest; gegebenenfalls halogen-, -alkoxy-($C_1$-$C_4$)- oder alkyl-($C_1$-$C_4$)-substituierte $C_6$-$C_{14}$-Arylreste wie der Phenyl- sowie der 1-Naphthyl- und der 2-Naphthyl-Rest; ferner racemische oder vorteilhafterweise optisch aktive Terpenylreste wie der Menthyl-, 8-Phenylmenthyl-, Neomenthyl-, Isomenthyl-, Bornyl-, Fenchyl-, Pinanyl- und Isopinocamphyl-Rest. Reste $R_2$ die chiral sind, können in racemischer Form oder vorteilhafterweise in optisch aktiver Form eingesetzt werden.

Von besonderem Interesse sind Verbindungen der allgemeinen Formel (I), in welcher

$R_1$ für Alkyl mit 1 bis 4 C-Atomen, Benzyl, Cycloalkyl mit 3 bis 7 C-Atomen, Phenyl, Indolylmethyl, Naphthyl, Naphthylmethyl, Furyl, Thienyl oder Pyridyl steht, wobei die genannten Alkyl- und Aryl-Reste gegebenenfalls ein- oder zweifach substituiert sind durch Hydroxy, Methoxy, Halogen, Alkyl, Cycloalkyl mit bis zu 6 C-Atomen, Phenoxy, Benzoxy, Acetylamino, Benzyloxycarbonyl oder Alkoxycarbonyl mit bis zu 4 C-Atomen,

$R_3$ für Wasserstoff, Methyl oder Ethyl steht oder zusammen mit $R_1$ eine Tri- oder Tetramethylengruppe bildet,

X für Sauerstoff oder eine $NR_4$-Gruppe steht, in welcher

$R_4$ für Wasserstoff oder Alkyl mit 1 bis 4 C-Atomen steht oder gemeinsam mit $R_2$ und dem Stickstoffatom einen 5- bis 7-gliedrigen Ring bildet, der gegebenenfalls durch Alkyl oder Alkoxycarbonyl mit jeweils bis zu 4 C-Atomen substituiert ist und

$R_2$ für Alkyl mit bis zu 12 C-Atomen, Benzyl, Cycloalkyl mit 3 bis 7 C-Atomen, Phenyl oder Terpenyl steht, wobei die genannten Reste gegebenenfalls substituiert sind durch Fluor, Chlor oder Brom oder durch Alkyl oder Alkoxy mit jeweils bis zu 2 C-Atomen.

Die erfindungsgemäßen optisch aktiven N-$\alpha$-Fluoracryloylaminosäure-Derivate der Formel (I) leiten sich vorzugsweise von optisch aktiven Aminosäuren wie Alanin, Aminobuttersäure, Valin, Norvalin, Leucin, Isoleucin, Terleucin, Phenylglycin, Naphthylglycin, Phenylalanin, Thienylalanin, Pyridylalanin, Naphthylalanin, Cyclohexylglycin, Cyclohexylalanin, Tyrosin, Tryptophan, Serin, Asparaginsäure, Glutaminsäure, Ornithin, Lysin, Prolin oder 6-Aminopenicillansäure ab, das heißt, $R^1$ steht vorzugsweise für Alkyl mit 1 bis 4 C-Atomen, Benzyl, Cyclohexyl, Phenyl, Indolylmethyl, Naphthyl, Naphthylmethyl, Thienyl oder Pyridyl, wobei die genannten Alkyl- und Aryl-Reste gegebenenfalls einfach substituiert sein können durch Hydroxy, Phenoxy, Benzoxy, Acetylamino, Benzyloxycarbonyl oder Alkoxycarbonyl mit bis zu 4 C-Atomen.

Die erfindungsgemäßen optisch aktiven N-Fluoracryloylaminosäure-Derivate der Formel (I) werden erhalten entweder

A) durch Umsetzung von optisch aktiven Aminosäure-Derivaten der Formel

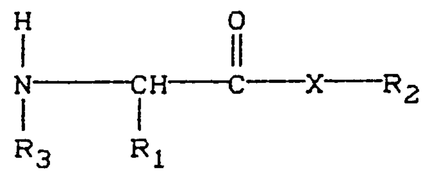

(II)

in der
$R_1$, $R_2$ und $R_3$ die unter Formel (I) angegebene Bedeutung haben,
oder deren Säureadditionsprodukten
mit Fluoracryloyl-Derivaten der Formel

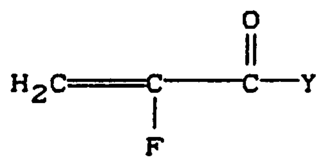

(III)

in der
Y für Fluor, Chlor oder Brom steht,
gegebenenfalls in Gegenwart eines Säurebindemittels in inerten organischen Lösungsmitteln
oder
B) durch Umsetzung von Verbindungen der Formel (II) mit einer Fluorverbindung der Formel

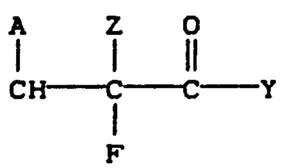

(IV)

in der

| | |
|---|---|
| Y | die oben angegebene Bedeutung hat, und |
| A und Z | jeweils für Wasserstoff, Fluor, Chlor oder Brom stehen, wobei A und Z nie gleichzeitig Wasserstoff bedeuten, so daß die Fluoracryloyl-Verbindung (III) durch AZ-Abspaltung freigesetzt werden kann, wobei AZ bevorzugt HF, HCl, HBr, $Br_2$ oder $Cl_2$ bedeutet. |

$\alpha$-Fluoracryloyl-Derivate der Formel (III) oder deren Vorstufen (IV) können nach an sich bekannten Verfahren hergestellt werden [Zh. Org. Khim. 28, 1173 (1983)] und gegebenenfalls auch als Säureanhydride eingesetzt werden.

Die als Ausgangsverbindungen verwendeten optisch aktiven Aminosäureester der Formel (II) sind bekannt oder können nach an sich bekannten Verfahren hergestellt werden (siehe Bull. Chem. Soc. Jap. 37 (1964), 191).

Geeignete Säureadditionsverbindungen der als Ausgangsverbindungen zu verwendenden Aminosäuren sind Salze dieser Aminosäuren mit anorganischen oder organischen Säuren. Bevorzugt sind Mineralsäuren wie Salzsäure, Bromwasserstoffsäure, Schwefelsäure, Phosphorsäure oder organische Säuren wie Essigsäure, Methan-, Ethan-, Benzol- oder Toluolsulfonsäure.

Als Lösungsmittel eignen sich alle unter den Reaktionsbedingungen inerten organischen Lösungsmittel. Bevorzugt sind Kohlenwasserstoffe wie Benzol, Toluol, Xylol oder Erdölfraktionen, oder Halogenkohlenwasserstoffe wie Di-, Tri- oder Tetrachlormethan, Dichlorethan oder Trichlorethylen oder Ether wie tert.-Butylmethylether oder tert.-Amyl-ethylether.

Als Säurebindemittel kommen vor allem die üblichen anorganischen oder organischen Basen in Betracht; bevorzugt werden Alkali- oder Erdalkalihydroxide wie Natrium-, Kalium-, Lithium-, Calcium- oder

Bariumhydroxid, Alkali- oder Erdalkalicarbonate wie Natrium- oder Kaliumcarbonat oder Natriumbicarbonat, Alkalialkoholate wie Natriummethylat, -ethylat, Kaliummethylat, -ethylat, -t-butylat oder Amine wie Triethylamin oder Pyridin, verwendet.

Die Umsetzung der α-Fluoracryloyl-Derivate der Formel (III) mit den Aminosäure-Derivaten der Formel (II) wird bevorzugt bei Temperaturen von -78 bis +100 °C, insbesondere von -20 °C bis +60 °C vorgenommen.

Die Erfindung betrifft auch die durch Polymerisation bzw. Copolymerisation der optisch aktiven N-α-Fluoracryloylaminosäure-Derivate der Formel (I) erhältlichen optisch aktiven Polymere und Copolymere, die mindestens 40 Mol-%, vorzugsweise mindestens 50 Mol-% Struktureinheiten der Formel (V)

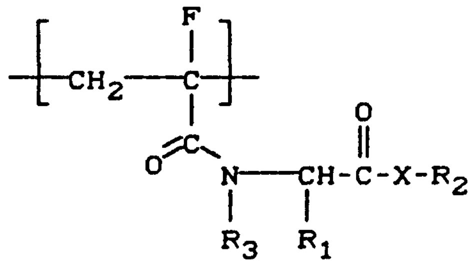

(V)

enthalten,
in der $R_1$, $R_2$, $R_3$ und X die unter Formel (I) angegebene Bedeutung haben.

Die erfindungsgemäßen optisch aktiven Polymere der Formel (V) liegen vorzugsweise in Form von vernetzten unlöslichen oder quellbaren Perl-Polymerisaten oder in an feinteilige anorganische Trägermaterialien wie z.B. Kieselgel gebundener Form vor. Sie können auch als lineare, in geeigneten organischen Lösungsmitteln lösliche Polymere hergestellt werden. Es ist ferner möglich, verschiedene erfindungsgemäße N-α-Fluoracryloylaminosäure-Derivate der Formel (I) cozupolymerisieren, sowie 0,1 bis 60, vorzugsweise 0,1 bis 20 Mol-% copolymerisierbarer, anderer Monomere in das Polymere einzubauen.

Die vernetzten Polymerisate liegen vorzugsweise in Form kleiner Teilchen (Perlen) mit 5 bis 200 µm Teilchendurchmesser vor. Sie werden z.B. durch Suspensionspolymerisation der optisch aktiven N-α-Fluoracryloylaminosäure-Derivate der Formel (I) mit 0,5 bis 50 Mol-%, vorzugsweise 1 bis 30 Mol-%, besonders bevorzugt 3 bis 20 Mol-% (bezogen auf die Gesamtmenge [Mol] der eingesetzten Monomere) eines geeigneten Vernetzers in an sich bekannter Weise hergestellt.

Durch die Art und Menge der Vernetzer läßt sich der Quellungsgrad der (Perl)Polymerisate nach üblichen Methoden einstellen.

Bei der praktischen Verwendung haben sich (Perl)Polymerisate mit einem Quellungsgrad (Q) von 1.1 bis 10, bevorzugt von 2.0 bis 7.0 bewährt.

Der Quellungsgrad Q wird wie folgt bestimmt:

$$Q = \frac{\text{Polymerisatvolumen (gequollen)}}{\text{Polymerisatvolumen (ungequollen)}}$$

Als Vernetzungsmittel kommen Verbindungen in Frage, die mindestens zwei polymerisierbare Vinylgruppen enthalten. Bevorzugte Vernetzungsmittel sind Alkandioldiacrylate wie 1,6-Hexandioldiacrylat, 1,4-Butandioldiacrylat, 1,3-Propandioldiacrylat oder 1,2-Ethylenglykoldiacrylat, oder Alkandiolmethacrylate wie 1,4-Butandioldimethacrylat, 1,3-Butandioldimethacrylat, 2,3-Butandioldimethacrylat, 1,3-Propandioldimethacrylat oder 1,2-Ethylenglykoldimethacrylat, aromatische Divinylverbindungen wie beispielsweise Divinylbenzol, Divinylchlorbenzol oder Divinyltoluol, Dicarbonsäurevinylester wie Adipinsäuredivinylester, Benzoldicarbonsäuredivinylester, Terephthalsäuredivinylester, N,N'-Alkylendiacrylamide wie N,N'-Methylendiacrylamid, N,N'-Ethylendiacrylamid, N,N'-Methylendimethacrylamidoder N,N'-Ethylendimethacrylamid.

Als Radikalbildner kommen die üblichen Radikalbildner in Frage. Bevorzugt sind Peroxide wie beispielsweise Dibenzoylperoxid, Dilauroylperoxid oder Di-orthotolylperoxid oder Azoverbindungen wie beispielsweise Azobisisobuttersäurenitril (AIBN). Auch Gemische verschiedener Radikalbildner sind verwendbar.

Die Polymerisationskomponenten werden in einem nicht mit Wasser mischbaren organischen Lösungsmittel, vorzugsweise einem aliphatischen oder aromatischen Kohlenwasserstoff wie Hexan, Heptan, Isododecan, Benzol oder Toluol, einem Halogenkohlenwasserstoff wie Di-, Tri-, Tetrachlormethan oder 1,2-Dichlorethan oder einem Ester wie Essigsäureethylester bzw. Essigsäurebutylester gelöst.

Die organische Phase wird mit Hilfe eines wirksamen Rührers in der wäßrigen Lösung eines Schutzkolloids, bevorzugt in einer wäßrigen Lösung von Polyvinylalkohol, Polyvinylpyrrolidon oder eines Copolymers aus Methacrylsäure und Methylmethacrylat, gleichmäßig verteilt. Je Gewichtsteil organische Phase verwendet man etwa 1 bis 20, bevorzugt 2 bis 10 Gew.-Teile wäßrige Phase. Das Polymerisationsgemisch wird unter Rühren in einer Inertgasatmosphäre, vorzugsweise unter Stickstoff, auf Temperaturen von 30 °C bis 150 °C, vorzugsweise 40 °C bis 80 °C erhitzt. Die Polymerisationsdauer beträgt zwischen 2 und 24, bevorzugt 4 und 12 Stunden. Das auf diese Weise erhaltene Copolymerisat wird durch Filtration von der flüssigen Phase getrennt, durch gründliches Waschen mit Wasser und mit organischen Lösungsmitteln wie Methanol, Ethanol, Benzol, Toluol, Di-, Trichlormethan oder Aceton gereinigt und anschließend getrocknet.

Insbesondere für analytische Anwendungen werden die erfindungsgemäßen optisch aktiven Polymere vorzugsweise in an feinteilige anorganische Träger gebundener Form eingesetzt. Die Herstellung solcher optisch aktiver Chromatographiephasen kann beispielsweise nach den in DE-A 3 706 890 beschriebenen Verfahren erfolgen.

Bevorzugt ist die Polymerisation der optisch aktiven N-α-Fluoracryloylaminosäure-Derivate der Formel (I) in Gegenwart von Vinylkieselgelen oder von Kieselgel-Diolphasen, die mit (Meth)acrylsäure verestert wurden. Die Polymerisation kann dabei in Abwesenheit von Lösungsmitteln oder in Gegenwart von Lösungsmitteln oder von Fällungsmitteln für die Poly-N-α-fluoracrylamide durchgeführt werden. Als Initiatoren können die zur Herstellung der Perlpolymerisate verwendeten Radikalbildner ebenfalls eingesetzt werden.

Die polymermodifizierten Kieselgele enthalten vorzugsweise 1 bis 40 Gew.-%, insbesondere 5 bis 30 Gew.-% an optisch aktivem Polymer (V), bezogen auf das Gesamtgewicht. Sie werden intensiv mit Lösungsmitteln für das Polymere gewaschen und im Vakuum getrocknet.

Selbstverständlich können auch hier Gemische von zwei oder mehreren der erfindungsgemäßen N-α-Fluoracryloylaminosäure-Derivate, gegebenenfalls auch mit weiteren copolymerisierbaren Monomeren eingesetzt werden.

Die Erfindung betrifft ferner die Verwendung der erfindungsgemäßen Poly-N-α-fluoracrylamide der Formel (V) als solche oder in vernetzter bzw. an Kieselgel gebundener Form zur chromatographischen Trennung von racemischen Gemischen in die optischen Antipoden. Gut trennbare Racemate sind beispielsweise Oxazepam, Binaphthol, Benzoin, Chlorthalidon, Thalidomid, N-3,5-Dinitrobenzoylleucin, 1-(9-Anthryl)-2,2,2-trifluorethanol sowie Tetrahydro- bzw. Hexahydrocarbazolderivate wie z.B. 3-(4-Chlorphenylsulfonamido)-9-(2-carboxyethyl)-1,2,3,4-tetrahydrocarbazol, 3-r-(Benzol-sulfonamido)-9-(2-carboxyethyl)-1,2,3,4,4a,9a-hexahydrocarbazol, 1-(Carboxymethyl)-6-fluor-9-(4-chlorbenzyl)-1,2,3,4-tetrahydrocarbazol, Hydroxyalkylazolderivate wie z.B. 2-(4-Chlorphenyl)-3-methoxyimino-3-methyl-1-(1,2,4-triazolyl-1)-2-butanol und α-(4-Fluorphenyl)-α-(1-cyancyclopropyl)-1H-(1,2,4-triazolyl-1)-ethanol und 1,4-Dihydropyridin-Derivate wie z.B. 1,4-Dihydro-2,6-dimethyl-4-(2-trifluormethylphenyl)-3,5-dicarbonsäure-5-ethylester.

Die Zusammensetzung des Fließmittels kann je nach Art und Eigenschaft des zu trennenden Racemates in üblicher Weise ausgewählt und optimiert werden. Die an Kieselgel gebundenen erfindungsgemäßen Poly-N-α-fluoracryloylaminosäurederivate können für chromatographische Racemattrennungen unter HPLC-Bedingungen eingesetzt werden.

Die Fähigkeit der Polymerisate zur Racemattrennung wird durch die Kapazitätsverhältnisse ($k'_{1(2)}$-Werte) für die beiden Enantiomere (1) und (2) und den daraus resultierenden Enantioselektivitätswert $\alpha$ ausgedrückt. Diese chromatographischen Parameter sind wie folgt definiert:

$$\text{Kapazitätsverhältnis } k_{1(2)} = \frac{t_{1(2)} - t_0}{t_0}$$

$$\text{Enantioselektivität } \alpha = \frac{k'_2}{k'_1}$$

$t_o$ = Totzeit der Säule

$t_{1(2)}$ = Retentionszeit des zuerst eluierten Enantiomers 1 bzw. des später eluierten Enantiomers 2

Die präparative Trennung von racemischen Gemischen in ihre optischen Antipoden unter Verwendung der erfindungsgemäßen Polymerisate wird vorzugsweise säulenchromatographisch vorgenommen. Besonders vorteilhaft ist es hierfür, die chromatographische Trennung mit Perlpolymerisaten einer bestimmten Korngrößenverteilung vorzunehmen; gute Trennleistungen werden mit Perlpolymerisaten einer Korngrößenverteilung von 5 bis 200 µm, bevorzugt 15 bis 100 µm erhalten.

Die Arbeitsmethodik der säulenchromatographischen Trennung ist bekannt. Üblicherweise wird das Polymerisat im Fließmittel suspendiert und die Suspension in eine Glassäule gefüllt. Nach Ablaufen des Fließmittels wird das zu trennende Racemat, gelöst in möglichst wenig Fließmittel, auf die Säule aufgebracht. Dann wird mit Fließmittel eluiert und die Enantiomeren im Eluat photometrisch und/oder polarimetrisch mittels geeigneter Durchflußzellen detektiert.

Als Fließmittel werden übliche organische Lösungsmittel bzw. Lösungsmittelgemische verwendet, die das als Adsorbens eingesetzte Polymerisat anquellen und das zu trennende Racemat lösen. Beispielsweise seien genannt: Kohlenwasserstoffe wie Benzol, Toluol oder Xylol, Ether wie Diethylether, t-Butylmethylether, Dioxan oder Tetrahedronfuran, Halogenkohlenwasserstoffe wie Di- oder Trichlormethan, Aceton, Acetonitril oder Essigester, Alkohole wie Methanol, Ethanol, n-Propanol, Isopropanol oder n-Butanol, oder aber Gemische der genannten Lösungsmittel. Als besonders geeignet haben sich Mischungen aus Toluol mit Tetrahydrofuran, Dioxan oder Isopropanol erwiesen.

## Beispiele

### I. Herstellungsmethode der N-α-Fluoracryloylaminosäurederivate (Monomere)

Methode a) ausgehend von 2,3-Difluorpropionsäurechlorid

Man legt eine Lösung von 0,2 Mol 2,3-Difluorpropionylchlorid in 200 ml abs. Dichlormethan vor, tropft bei 0 °C 0,205 Mol Triethylamin zu und rührt 30 Minuten bei 0 °C nach.

Zu dieser Lösung tropft man dann bei -10 °C eine Mischung aus 0,2 Mol Aminosäure-Derivat und 0,2 Mol Triethylamin in 200 ml abs. Dichlormethan.

Man läßt auf Raumtemperatur kommen und arbeitet dann wie üblich wäßrig auf.

Methode b) unter Verwendung von α-Fluoracrylsaurechlorid

Zu einer Lösung von 0,1 Mol eines Aminosäureesters bzw. -amidhydrochlorids der Formel (II) in 300 ml Dichlormethan tropft man bei 0 °C zunächst 0,2 Mol Triethylamin und anschließend bei -10 °C eine Lösung von 0,1 Mol α-Fluoracrylsäurechlorid in 25 ml Dichlormethan. Man rührt 30 Minuten ohne Kühlbad nach, wäscht sukzessive mit Wasser, 1N HCl und gesättigter $NaHCO_3$-Lösung, trocknet über Magnesiumsulfat und befreit am Rotationsverdampfer vom Solvens. Das nach Trocknen im Hochvakuum erhaltene Produkt kristallisiert man um oder reinigt durch Chromatographie an Kieselgel.

Setzt man einen freien Aminosäureester bzw. -amid ein, so reduziert man die Triethylamin-Menge auf die Hälfte. Die so hergestellten N-α-Fluoracryloylaminosäurederivate sind in der Tabelle I zusammengestellt.

Tabelle I optisch aktive Monomere (I)

| Beispiel Nr. | N-α-Fluoracryloylaminosäure-derivat | Herstell-methode | Fp ($^{o}$C) | Drehwert (c=1, $CHCl_3$) | Ausbeute (% d.Th.) |
|---|---|---|---|---|---|
| 1 | N-Fluoracryloyl-L-phenylalanin-ethylester | a | 36$^{o}$ C | +112$^{o}$ | 65 |
| 2 | N-Fluoracryloyl-L-phenylalanin-n-propylester | a | 31$^{o}$ C | +103$^{o}$ | 73 |
| 3 | N-Fluoracryloyl-L-phenylalanin-i-propylester | a | 82$^{o}$ C | +106,9$^{o}$ | 78 |
| 4 | N-Fluoracryloyl-L-phenylalanin-t-butylester | b | 71$^{o}$ C | +103,2$^{o}$ | 89 |
| 5 | N-Fluoracryloyl-L-phenylalanin d-menthylester | b | Öl | +85,4$^{o}$ | 63 |
| 6 | N-Fluoracryloyl-L-alanin-d-menthylester | b | 34$^{o}$ C | +55,8$^{o}$ | 83 |
| 7 | N-Fluoracryloyl-L-alanin-l-bornylester | b | 80$^{o}$ C | -38,1$^{o}$ | 80 |
| 8 | N-Fluoracryloyl-L-alanin-(+)-fenchylester | b | Öl | +36,4$^{o}$ | 83 |
| 9 | N-Fluoracryloyl-L-leucin-t-butylester | b | 47$^{o}$ C | +3,1$^{o}$ | 83 |
| 10 | N-Fluoracryloyl-D-phenylglycin-t-butylester | b | 117$^{o}$ C | -176,8$^{o}$ | 64 |

II. Polymerisation der N-α-Fluoracryloylaminosäure-Derivate (I)

1. Herstellung in an Kieselgel gebundener Form

a) 25 g mit 1,2-Diolgruppen modifiziertes Kieselgel (mittlere Korngröße: 5 µm) werden unter Feuchtigkeitsausschluß und unter Stickstoff in 500 ml Dioxan suspendiert. Die Suspension wird mit 16 ml Methacrylsäureanhydrid und 12,5 ml Triethylamin versetzt. Die Mischung wird 1 Stunde bei Raumtemperatur gerührt und 24 Stunden bei Raumtemperatur aufbewahrt. Dann wird das Kieselgel über eine Glasfritte (G4) abgesaugt, 3 mal mit je 500 ml Dioxan 30 Minuten lang verrührt und zwischendurch gut trockengesaugt. Das durch Methacryloylgruppen modifizierte Kieselgel wird bei Raumtemperatur im Vakuum <0,005 at getrocknet.
Ausbeute 24,8 g
Elementaranalyse: C: 9,2 %; H: 1,7 %
Werte des mit Diolgruppen modifizierten Kieselgels:
C: 7,7 %; H: 1,5 %

b) In einem mit Rückflußkühler und Magnetrührer versehenen 100 ml-Rundkolben werden 3 g des mit Methacryloylgruppen modifizierten Kieselgels, dessen Herstellung unter a) beschrieben ist, 6,0 g optisch aktives N-α-Fluoracryloyl-aminosäure-Derivat und 60 mg Azobisisobutyronitril in 25 ml trockenem Toluol gelöst bzw. suspendiert. Die Apparatur wird durch dreimaliges Evakuieren und mit Stickstofffüllen von Luft befreit und anschließend mit Stickstoff gefüllt. Das Polymerisationsgemisch wird 1 Stunde bei Raumtemperatur gerührt und dann rasch auf 80 °C erhitzt. Nach 45-minütigem Rühren bei 80 °C werden 200 mg 2,6-Di-tert.-butyl-4-methylphenol zugesetzt und das Reaktionsgemisch rasch abgekühlt. Das Kieselgel wird über eine Glasfritte (G4) abgesaugt, mit Toluol gewaschen und 2 mal mit je 50 ml Chloroform, einmal mit 50 ml Toluol und einmal mit 50 ml Isopropanol je 30 Minuten verrührt und zwischendurch abgesaugt. Das Kieselgel wird abschließend bei Raumtemperatur im Vakuum <0,005 at getrocknet. In der nachstehenden Tabelle II sind die auf das modifizierte Kieselgel aufpolymerisierten N-α-Fluoracryloyl-aminosäure-Derivate, die Ausbeuten an optisch aktive Verbindungen enthaltendem Kieselgel, dessen Stickstoffgehalt und dessen Gehalt an gebundenem Polymer zusammengestellt.

c) In der unter b) beschriebenen Apparatur werden 3,0 g des mit Methacryloylgruppen modifizierten Kieselgels, dessen Herstellung unter a) beschrieben ist, 6,0 g optisch aktives N-α-Fluoracryloyl-aminosäure-Derivat und 60 mg Azobisisobutyronitril in 25 ml trockenem Trichlormethan gelöst bzw. suspendiert. Die Apparatur wird durch dreimaliges abwechselndes Evakuieren und mit Stickstoffüllen von Luft befreit und abschließend mit Stickstoff gefüllt. Das Reaktionsgemisch wird 1 Stunde bei Raumtemperatur und 1 Stunde unter Rückfluß gerührt. Nach Zugabe von 200 mg 2,6-Di-tert.-butyl-4-methylphenol wird der Ansatz rasch abgekühlt. Das Kieselgel wird über eine Glasfritte (G4) abgesaugt, zweimal mit je 50 ml Chloroform, einmal mit 50 ml Toluol und einmal mit 50 ml Isopropanol je 30 Minuten verrührt und zwischendurch abgesaugt. Das Kieselgel wird abschließend bei Raumtemperatur im Vakuum <0,005 at getrocknet.

<u>Tabelle II</u> optisch aktive Polymere (V) an Kieselgel

| Bsp. Nr. | N-α-Fluoracryloylaminosäure-Derivat gemäß Bsp. | Herstellungsvorschrift | Ausbeute (g) | [N-Gehalt] (%) | Gehalt des Kieselgels an gebundenem Polymerisat (Gew.-%) |
|---|---|---|---|---|---|
| 11 | 1 | 1b | 3,3 | 1,0 | 18,9 |
| 12 | 2 | 1c | 3,3 | 0,75 | 15,0 |
| 13 | 3 | 1b | 3,15 | 0,7 | 14,0 |
| 14 | 4 | 1b | 3,35 | 0,75 | 15,7 |
| 15 | 5 | 1b | 3,15 | 0,55 | 14,8 |
| 16 | 6 | 1b | 3,1 | 0,65 | 13,9 |
| 17 | 7 | 1b | 3,25 | 0,75 | 15,9 |
| 18 | 8 | 1b | 3,4 | 0,65 | 13,8 |
| 19 | 9 | 1b | 3,05 | 0,7 | 13,0 |
| 20 | 10 | 1b | 3,1 | 0,9 | 18,0 |

2. <u>Herstellung in Form von Perlpolymerisaten</u>

Die Lösung von 13,5 g optisch aktivem N-α-Fluoracryloylaminosäure-Derivat, 1,5 g Ethylenglykoldimethacrylat und 0,3 g Azobisisobutyronitril in 37,5 g Trichlormethan wird unter Rühren (350 bis 500 U/min) in

einer Lösung von 3 g Polyvinylalkohol in 130 ml entmineralisiertem Wasser dispergiert. Die Apparatur wird mehrmals evakuiert und mit Stickstoff gefüllt. Das Polymerisationsgemisch wird unter Stickstoff zunächst 30 Minuten bei Raumtemperatur und anschließend 16 Stunden bei 55 °C (Innentemperatur) gerührt. Das Polymerisationsgemisch wird dann in 2 bis 3 l Wasser eingerührt und die flüssige Phase nach dem Absitzen des Perlpolymerisates dekantiert. Das Perlpolymerisat wird durch 3 bis 4-maliges Suspendieren in Wasser und Abdekantieren der flüssigen Phase vom Feinkorn (Polymerisat mit einer Korngröße von <10 µm) befreit und nach intensivem Waschen mit Aceton bei 60 °C bis zur Gewichtskonstanz getrocknet.

In der nachstehenden Tabelle III sind die für die Polymerisation verwendeten N-α-Fluoracryloylaminosäure-Derivate, die Rührgeschwindigkeit, bei der die Polymerisation durchgeführt wurde, die Ausbeuten, in denen die Polymerisate erhalten wurden, deren Korngröße und das Volumen der erhaltenen Perlpolymerisate in trockenem (Vs) und gequollenem (Vq) Zustand (Quellmittel: Toluol = T bzw. Toluol/THF 3:2 v/v-Gemisch = T/T) zusammengestellt.

**Tabelle III** optisch aktive Polymere (V) als Perlpolymerisate

| Bsp. Nr. | N-Fluoracryloylaminosäure-Derivat gemäß Beispiel | Rührgeschwindigkeit [U/min] | Ausbeute an Perlen >10 µm (g) | Korngröße der Perlen (µm) | $V_s$ (ml/g) | $V_q$ (ml/g) | Quellmittel |
|---|---|---|---|---|---|---|---|
| 21 | 1 | 500 | 9,8 | 15-80 | 1,8 | 5,5 | T |
| 22 | 2 | 450 | 9,2 | 15-70 | 2,1 | 5,7 | T |
| 23 | 3 | 350 | 11,7 | 20-120 | 1,6 | 5,5 | T |
| 24 | 4 | 400 | 12,3 | 20-110 | 1,6 | 5,2 | T |
| 25 | 5 | 400 | 12,1 | 15-80 | 1,6 | 6,0 | T/T |
| 26 | 6 | 400 | 12,4 | 15-80 | 1,7 | 5,7 | T |
| 27 | 7 | 450 | 11,8 | 15-85 | 2,3 | 5,5 | T/T |
| 28 | 8 | 450 | 11,6 | 15-80 | 1,8 | 5,6 | T/T |
| 29 | 9 | 450 | 12,2 | 15-75 | 1,9 | 6,3 | T/T |
| 30 | 10 | 450 | 12,0 | 20-110 | 1,6 | 4,8 | T |

III. Verwendung der N-α-Fluoracryloylaminosäure-Derivate (I) als Adsorbentien für die Racemattrennung

Für die chromatographischen Trennungen wurden folgende Testracemate verwendet:
Racemat Nr. 1: Oxazepam

Racemat Nr. 2: Binaphthol
Racemat Nr. 3: Chlorthalidon
Racemat Nr. 4: Thalidomid
Racemat Nr. 5: N-3,5-Dinitrobenzoylleucin
Racemat Nr. 6: 3-r-(Benzol-sulfonamido)-9-(2-carboxyethyl)-1,2,3,4,4a,9a-hexahydrocarbazol

Die an Kieselgel gebundenen Polymerisate wurden in Stahlsäulen (Innendurchmesser: 4 mm, Länge: 25 cm) eingesetzt. Eluiert wurde mit n-Heptan-Tetrahydrofuran-Gemischen 3:2 = Elutionsmittel a, 1:1 = Elutionsmittel b, 1:2 = Elutionsmittel c; die Fließmittelgeschwindigkeit betrug 1 ml/min.

Die nachstehenden Chromatogramme zeigen die überraschend gute Trennwirkung der erfindungsgemäßen Polymeren. Die Abszisse gibt die Elutionszeit in Minuten an. Die Detektion erfolgt durch UV-Absorption.

Im Vergleich zu den erfindungsgemäßen Polymeren wurde versucht, an einer mit N-Acryloyl-phenylalaninethylester belegten Kieselgelsäule (Chiraspher® von Merck) die Trennung der Testracemate 2 und 4 im Elutionsmittel a und des Testracemats 3 im Elutionsmittel b durchzuführen. In allen Fällen wurde bei UV-Detektion keine Enantiomerentrennung beobachtet, die Enantioselektivität $\alpha$ betrug 1,00, obwohl das eingesetzte Polymer sich nur durch die fehlende Fluorsubstitution vom Polymer des Beispiels 11 unterscheidet.

Abbildung 1
Racemat Nr. 1
Adsorbens gemäß Beispiel 17
Elutionsmittel: a

Abbildung 2
Racemat Nr. 2
Adsorbens gemäß Beispiel 11
Elutionsmittel: a

Abbildung 3
Racemat Nr. 2
Adsorbens gemäß Beispiel 16
Elutionsmittel: a

Abbildung 4
Racemat Nr. 3
Adsorbens gemäß Beispiel 11
Elutionsmittel: b

Abbildung 5
Racemat Nr. 4
Adsorbens gemäß Beispiel 11
Elutionsmittel: c

Abbildung 6
Racemat Nr. 4
Adsorbens gemäß Beispiel 18
Elutionsmittel: a

Abbildung 7
Racemat Nr. 5
Adsorbens gemäß Beispiel 17
Elutionsmittel: a

Abbildung 8
Racemat Nr. 6
Adsorbens gemäß Beispiel 16
Elutionsmittel: a

Abbildung 9
Racemat Nr. 6
Adsorbens gemäß Beispiel 18
Elutionsmittel: a

**Patentansprüche**

**Patentansprüche für folgende Vertragsstaaten : AT, BE, CH, DE, DK, FR, GB, IT, LI, LU, NL, SE**

1. Optisch aktive N-α-Fluoracryloylaminosäurederivate der Formel (I)

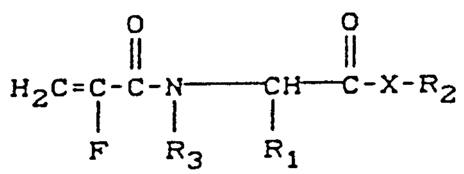

(I),

in der

$R_1$ für einen geradkettigen oder verzweigten $C_1$-$C_8$-Alkyl-, einen $C_7$-$C_{12}$-Aralkyl-, einen $C_3$-$C_{10}$-Cycloalkyl-, einen $C_6$-$C_{14}$-Aryl- oder einen Furyl-, Thienyl- oder Pyridylrest steht, die gegebenenfalls durch Benzyloxycarbonyl, Alkoxycarbonyl mit bis zu 6 C-Atomen, Hydroxyl, Alkyl, Cycloalkyl oder Alkoxy mit jeweils bis zu 6 C-Atomen, Halogen, Phenoxy, Benzoxy, Acylamino mit bis zu 8 C-Atomen oder durch Carbonylalkoxy mit bis zu 6 C-Atomen substituiert sind,

$R_3$ für Wasserstoff oder $C_1$-$C_4$-Alkyl steht oder zusammen mit $R_1$ eine Tri- oder Tetramethylengruppe bildet,

X Sauerstoff oder eine $NR_4$-Gruppe bedeutet, in der

$R_4$ für Wasserstoff oder geradkettiges oder verzweigtes $C_1$-$C_4$-Alkyl steht oder zusammen mit $R_2$ und dem Stickstoffatom einen 5- bis 7-gliedrigen Ring bildet, der gegebenenfalls durch eine Alkoxycarbonylgruppe mit bis zu 6 Kohlenstoffatomen oder durch ein oder zwei Alkylgruppen mit je 1 bis 4 Kohlenstoffatomen substituiert ist,

$R_2$ einen geradkettigen oder verzweigten $C_1$-$C_{22}$-Alkyl-, $C_7$-$C_{12}$-Aralkyl-, $C_3$-$C_{10}$-Cycloalkyl-, $C_6$-$C_{14}$-Aryl-oder einen Terpenylrest darstellt, die gegebenenfalls substituiert sind durch Halogen, Alkyl oder Alkoxy mit jeweils 1 bis 4 C-Atomen.

2. Verbindungen der allgemeinen Formel (I) gemäß Anspruch 1, in welcher

$R_1$ für Alkyl mit 1 bis 4 C-Atomen, Benzyl, Cycloalkyl mit 3 bis 7 C-Atomen, Phenyl, Furyl, Indolylmethyl, Naphthyl, Naphthylmethyl, Thienyl oder Pyridyl steht, wobei die genannten Alkyl- und Aryl-Reste gegebenenfalls ein- oder zweifach substituiert sind durch Hydroxy, Methoxy, Halogen, Alkyl, Cycloalkyl mit bis zu 6 C-Atomen, Phenoxy, Benzoxy, Acetylamino, Benzyloxycarbonyl oder Alkoxycarbonyl mit bis zu 4 C-Atomen,

$R_3$ für Wasserstoff, Methyl oder Ethyl steht oder zusammen mit $R_1$ eine Tri- oder Tetramethylengruppe bildet,

X für Sauerstoff oder eine $NR_4$-Gruppe steht, in welcher

$R_4$ für Wasserstoff oder Alkyl mit 1 bis 4 C-Atomen steht oder gemeinsam mit $R_2$ und dem Stickstoffatom einen 5- bis 7-gliedrigen Ring bildet, der gegebenenfalls durch Alkyl oder Alkoxycarbonyl mit jeweils bis zu 4 C-Atomen substituiert ist und

$R_2$ für Alkyl mit bis zu 12 C-Atomen, Benzyl, Cycloalkyl mit 3 bis 7 C-Atomen, Phenyl oder Terpenyl steht, wobei die genannten Reste gegebenenfalls substituiert sind durch Fluor, Chlor oder Brom oder durch Alkyl oder Alkoxy mit jeweils bis zu 2 C-Atomen.

3. Verbindungen der allgemeinen Formel (I) gemäß Anspruch 1, dadurch gekennzeichnet, daß sie die Aminosäuresequenz der optisch aktiven Aminosäuren Alanin, Aminobuttersäure, Valin, Norvalin, Leucin, Isoleucin, Terleucin, Phenylglycin, Naphthylglycin, Phenylalanin, Thienylalanin, Pyridylalanin, Naphthylalanin, Cyclohexylglycin, Cyclohexylalanin, Tyrosin, Tryptophan, Serin, Asparaginsäure, Glutaminsäure, Ornithin, Lysin, Prolin oder 6-Aminopenicillansäure enthalten.

4. Verfahren zur Herstellung von Verbindungen der allgemeinen Formel (I) gemäß Anspruch 1, dadurch gekennzeichnet, daß man

A) optisch aktive Aminosäure-Derivate der Formel (II)

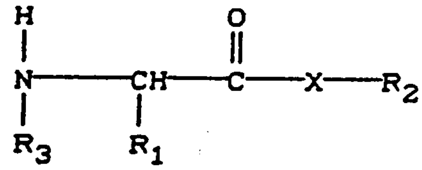

(II)

in der
$R_1$, $R_2$ und $R_3$ die unter Formel (I) angegebene Bedeutung haben,
oder deren Säureadditionsprodukten
mit Fluoracryloyl-Derivaten der Formel

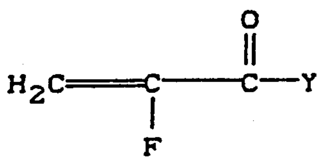

(III)

in der
Y für Fluor, Chlor oder Brom steht,
gegebenenfalls in Gegenwart eines Säurebindemittels in inerten organischen Lösungsmitteln umsetzt
oder
B) Verbindungen der Formel (II) mit einer Fluorverbindung der Formel (IV)

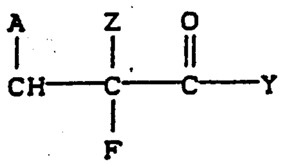

(IV)

in der
Y die oben angegebene Bedeutung hat, und
A und Z jeweils für Wasserstoff, Fluor, Chlor oder Brom stehen, wobei A und Z nie gleichzeitig Wasserstoff bedeuten,
unter Abspaltung AZ zu Fluoracrylverbindungen der Formel (III) umsetzt, gegebenenfalls in Gegenwart von inerten organischen Lösungsmitteln.

**5.** Optisch aktive Polymere, enthaltend mindestens 40 Mol-% von Struktureinheiten der Formel (V)

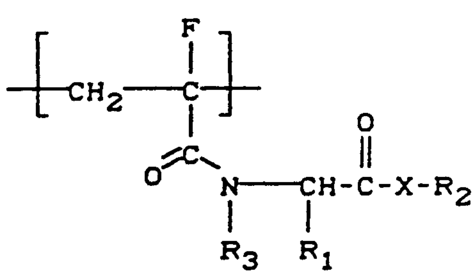

(V)

in welcher
R, $R_1$, $R_2$, $R_3$ und X die in Anspruch 1 angegebene Bedeutung haben.

**6.** Optisch aktive Polymere gemäß Anspruch 5, dadurch gekennzeichnet, daß sie in Form von vernetzten unlöslichen oder quellbaren Perlpolymerisaten oder in an feinteilige anorganische Trägermaterialien gebundener Form vorliegen.

**7.** Verfahren zur Herstellung von vernetzten Perlpolymerisaten gemäß Anspruch 6, dadurch gekennzeichnet, daß man die N-α-Fluoracryloyl-aminosäure-Derivate der allgemeinen Formel (I) mit 0,5 bis 50 Mol-% eines Vernetzers durch Suspensionspolymerisation, gegebenenfalls in Anwesenheit von Radikalbildnern und inerten organischen Lösungsmitteln herstellt.

**8.** Verfahren zur Herstellung von an anorganische Trägermaterialien gebundenen optisch aktiven Polymere gemäß Anspruch 6, dadurch gekennzeichnet, daß man Verbindungen der allgemeinen Formel (I) gemäß Anspruch 1 in Gegenwart von Vinylkieselgelen oder von gegebenenfalls (Meth)acryloyl-substituierten Kieselgel-Diolphasen gegebenenfalls in Anwesenheit von Radikalbildnern und gegebenenfalls in Anwesenheit von inerten organischen Lösungsmitteln verestert.

**9.** Verwendung von Polymeren gemäß Anspruch 7 zur Trennung von optischen Isomeren.

**Patentansprüche für folgende Vertragsstaaten : ES, GR**

**1.** Verfahren zur Herstellung von optischen aktiven N-α-Fluoracryloylaminosäurederivaten der Formel (I)

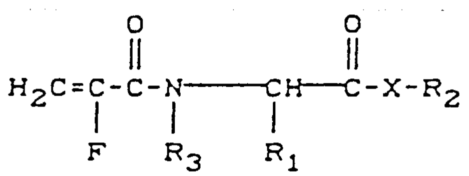

(I),

in der

$R_1$ für einen geradkettigen oder verzweigten $C_1$-$C_8$-Alkyl-, einen $C_7$-$C_{12}$-Aralkyl-, einen $C_3$-$C_{10}$-Cycloalkyl-, einen $C_6$-$C_{14}$-Aryl- oder einen Furyl-, Thienyl- oder Pyridylrest steht, die gegebenenfalls durch Benzyloxycarbonyl, Alkoxycarbonyl mit bis zu 6 C-Atomen, Hydroxyl, Alkyl, Cycloalkyl oder Alkoxy mit jeweils bis zu 6 C-Atomen, Halogen, Phenoxy, Benzoxy, Acylamino mit bis zu 8 C-Atomen oder durch Carbonylalkoxy mit bis zu 6 C-Atomen substituiert sind,

$R_3$ für Wasserstoff oder $C_1$-$C_4$-Alkyl steht oder zusammen mit $R_1$ eine Tri- oder Tetramethylengruppe bildet,

X Sauerstoff oder eine $NR_4$-Gruppe bedeutet, in der

$R_4$ für Wasserstoff oder geradkettiges oder verzweigtes $C_1$-$C_4$-Alkyl steht oder zusammen mit $R_2$ und dem Stickstoffatom einen 5- bis 7-gliedrigen Ring bildet, der gegebenenfalls durch eine Alkoxycarbonylgruppe mit bis zu 6 Kohlenstoffatomen oder durch ein oder zwei Alkylgruppen mit je 1 bis 4 Kohlenstoffatomen substituiert ist,

$R_2$ einen geradkettigen oder verzweigten $C_1$-$C_{22}$-Alkyl-, $C_7$-$C_{12}$-Aralkyl-, $C_3$-$C_{10}$-Cycloalkyl-, $C_6$-$C_{14}$-Aryl-oder einen Terpenylrest darstellt, die gegebenenfalls substituiert sind durch Halogen, Alkyl oder Alkoxy mit jeweils 1 bis 4 C-Atomen,

dadurch gekennzeichnet, daß man optisch aktive Aminosäure-Derivate der Formel (II)

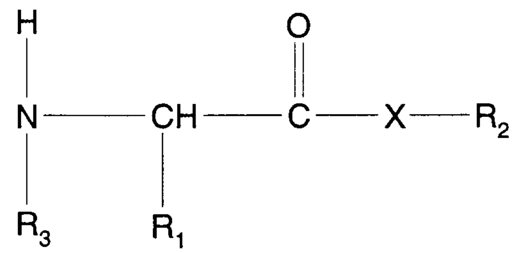

(II)

in der $R_1$, $R_2$ und $R_3$ die unter Formel (I) angegebene Bedeutung haben, oder deren Säureadditionsprodukten, mit Fluoracryloyl-Derivaten der Formel

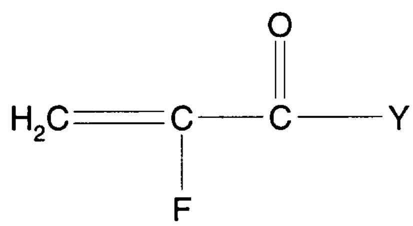

(III)

in der Y für Fluor, Chlor oder Brom steht, gegebenenfalls in Gegenwart eines Säurebindemittels in inerten organischen Lösungsmitteln umsetzt.

**2.** Verfahren zur Herstellung von Verbindungen nach Anspruch 1, dadurch gekennzeichnet, daß man Verbindungen der Formel (II) mit einer Fluorverbindung der Formel (IV)

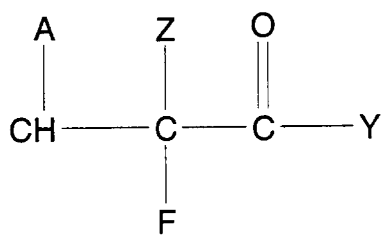

(IV)

in der

| | |
|---|---|
| Y | die oben angegebene Bedeutung hat, und |
| A und Z | jeweils für Wasserstoff, Fluor, Chlor oder Brom stehen, wobei A und Z nie gleichzeitig Wasserstoff bedeuten, |

unter Abspaltung AZ zu Fluoracrylverbindungen der Formel (III) umsetzt, gegebenenfalls in Gegenwart von inerten organischen Lösungsmitteln.

**3.** Verfahren zur Herstellung von optisch aktiven Polymeren, enthaltend mindestens 40 Mol-% von Struktureinheiten der Formel (V)

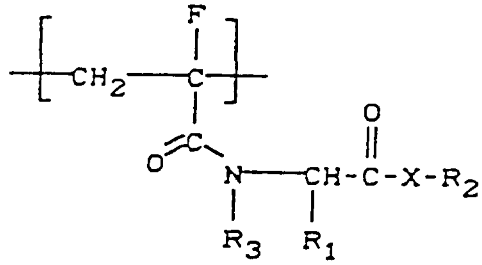

(V)

in welcher R, $R_1$, $R_2$, $R_3$ und X die in Anspruch 1 angegebene Bedeutung haben, dadurch gekennzeichnet, daß man die N-α-Fluoracryloyl-aminosäure-Derivate der allgemeinen Formel (I) mit 0,5 bis 50 Mol-% eines Vernetzers durch Suspensionspolymerisation, gegebenenfalls in Anwesenheit von Radikalbildnern und inerten organischen Lösungsmitteln herstellt.

## Claims

**Claims for the following Contracting States : AT, BE, CH, DE, DK, FR, GB, IT, LI, LU, NL, SE**

**1.** Optically active N-α-fluoroacryloylamino acid derivatives of the formula (I)

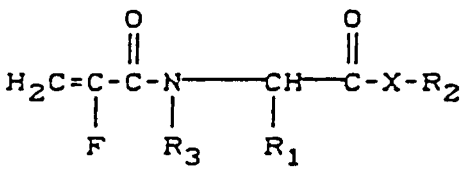

(I),

in which

$R_1$ represents a straight-chain or branched $C_1$-$C_8$-alkyl, a $C_7$-$C_{12}$-aralkyl, a $C_3$-$C_{10}$-cycloalkyl, a $C_6$-$C_{14}$-aryl or a furyl, thienyl or pyridyl radical, which are unsubstituted or substituted by benzyloxycarbonyl, alkoxycarbonyl of up to 6 C atoms, hydroxyl, alkyl, cycloalkyl or alkoxy each having up to 6 C atoms, halogen, phenoxy, benzoxy, acylamino of up to 8 C atoms or by carbonylalkoxy of up to 6 C atoms,

$R_3$ represents hydrogen or $C_1$-$C_4$-alkyl or together with $R_1$ forms a tri- or tetramethylene group,

X is oxygen or an $NR_4$ group, in which

$R_4$ represents hydrogen or straight-chain or branched $C_1$-$C_4$-alkyl or together with $R_2$ and the nitrogen atom forms a 5- to 7-membered ring which is unsubstituted or substituted by an alkoxycarbonyl group of up to 6 carbon atoms or by one or two alkyl groups each having 1 to 4 carbon atoms,

$R_2$ represents a straight-chain or branched $C_1$-$C_{22}$-alkyl, $C_7$-$C_{12}$-aralkyl, $C_3$-$C_{10}$-cycloalkyl, $C_6$-$C_{14}$-aryl or a terpenyl radical, each of which is unsubstituted or substituted by halogen, alkyl or alkoxy each having 1 to 4 C atoms.

**2.** Compounds of the general formula (I) according to Claim 1, in which

$R_1$ represents alkyl of 1 to 4 C atoms, benzyl, cycloalkyl of 3 to 7 C atoms, phenyl, furyl, indolylmethyl, naphthyl, naphthylmethyl, thienyl or pyridyl, in which the alkyl and aryl radicals mentioned are unsubstituted or mono- or disubstituted by hydroxyl, methoxy, halogen, alkyl, cycloalkyl of up to 6 C atoms, phenoxy, benzoxy, acetylamino, benzyloxycarbonyl or alkoxycarbonyl of up to 4 C atoms,

$R_3$ represents hydrogen, methyl or ethyl or together with $R_1$ forms a tri- or tetramethylene group,

X represents oxygen or an $NR_4$ group, in which

$R_4$ represents hydrogen or alkyl of 1 to 4 C atoms or together with $R_2$ and the nitrogen atom forms a 5-to 7-membered ring which is unsubstituted or substituted by alkyl or alkoxycarbonyl each having up to 4 C atoms, and

$R_2$ represents alkyl of up to 12 C atoms, benzyl, cycloalkyl of 3 to 7 C atoms, phenyl or terpenyl, in which the radicals mentioned are unsubstituted or substituted by fluorine, chlorine or bromine or by alkyl or alkoxy each having up to 2 C atoms

**3.** Compounds of the general formula (I) according to Claim 1, characterised in that they contain the amino acid sequence of the optically active amino acids alanine, aminobutyric acid, valine, norvaline, leucine, isoleucine, terleucine, phenylglycine, naphthylglycine, phenylalanine, thienylalanine, pyridylalanine, naphthylalanine, cyclohexylglycine, cyclohexylalanine, tyrosine, tryptophan, serine, aspartic acid, glutamic acid, ornithine, lysine, proline or 6-aminopenicillanic acid.

**4.** Process for the preparation of compounds of the general formula (I) according to Claim 1,

A) optically active amino acid derivatives of the formula (II)

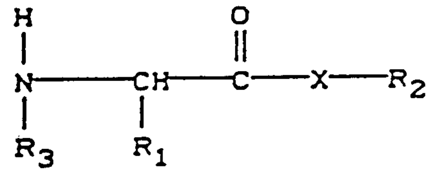

(II)

in which
$R_1$, $R_2$ and $R_3$ are as defined under formula (I),
or their acid addition products
are reacted with fluoroacryloyl derivatives of the formula

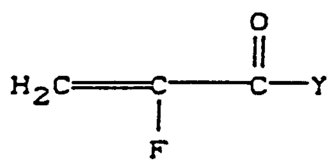

(III)

in which
Y represents fluorine, chlorine or bromine, if appropriate in the presence of an acid-binding agent in inert organic solvents, or
B) compounds of the formula (II) are reacted with a fluorine compound of the formula (IV)

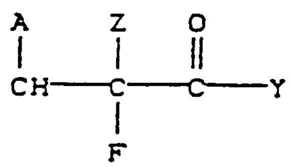

(IV)

in which
Y is as defined above, and
A and Z each represent hydrogen, fluorine, chlorine or bromine, in which A and Z never simultaneously denote hydrogen,
with elimination of AZ to give fluoroacrylic compounds of the formula (III), if appropriate in the presence of inert organic solvents.

**5.** Optically active polymers containing at least 40 mol% of structural units of the formula (V)

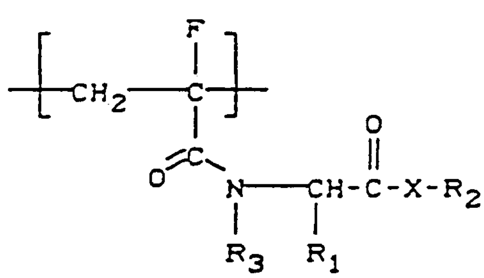

(V)

in which
R, $R_1$, $R_2$, $R_3$ and X are as defined in Claim 1.

6. Optically active polymers according to Claim 5, characterised in that they are present in the form of crosslinked insoluble or swellable bead polymers or in a form in which they are bound to finely divided inorganic support materials.

7. Process for the preparation of crosslinked bead polymers according to Claim 6, characterised in that the N-α-fluoroacryloylamino acid derivatives of the general formula (I) are prepared in the presence of 0.5 to 50 mol% of a crosslinking agent by suspension polymerisation, if appropriate in the presence of free-radical formers and inert organic solvents.

8. Process for the preparation of optically active polymers according to Claim 6, bound to inorganic support materials, characterised in that compounds of the general formula (I) according to Claim 1 are esterified in the presence of vinyl/silica gels or of unsubstituted or (meth)acryloyl-substituted silica gel/diol phases, if appropriate in the presence of free-radical formers and if appropriate in the presence of inert organic solvents.

9. Use of polymers according to Claim 7 for the resolution of optical isomers.

**Claims for the following Contracting States : ES, GR**

1. Process for the preparation of optically active N-α-fluoroacryloylamino acid derivatives of the formula (I)

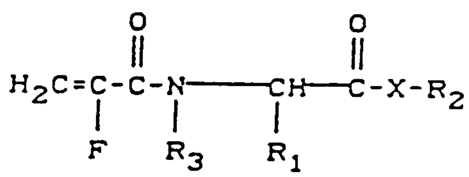

(I),

in which

$R_1$ represents a straight-chain or branched $C_1$-$C_8$-alkyl, a $C_7$-$C_{12}$-aralkyl, a $C_3$-$C_{10}$-cycloalkyl, a $C_6$-$C_{14}$-aryl or a furyl, thienyl or pyridyl radical, which are unsubstituted or substituted by benzyloxycarbonyl, alkoxycarbonyl of up to 6 C atoms, hydroxyl, alkyl, cycloalkyl or alkoxy each having up to 6 C atoms, halogen, phenoxy, benzoxy, acylamino of up to 8 C atoms or by carbonylalkoxy of up to 6 C atoms,

$R_3$ represents hydrogen or $C_1$-$C_4$-alkyl or together with $R_1$ forms a tri- or tetramethylene group,

X is oxygen or an $NR_4$ group, in which

$R_4$ represents hydrogen or straight-chain or branched $C_1$-$C_4$-alkyl or together with $R_2$ and the nitrogen atom forms a 5- to 7-membered ring which is unsubstituted or substituted by an alkoxycarbonyl group of up to 6 carbon atoms or by one or two alkyl groups each having 1 to 4 carbon atoms,

$R_2$ represents a straight-chain or branched $C_1$-$C_{22}$-alkyl, $C_7$-$C_{12}$-aralkyl, $C_3$-$C_{10}$-cycloalkyl, $C_6$-$C_{14}$-aryl or a terpenyl radical, each of which is unsubstituted or substituted by halogen, alkyl or alkoxy each having 1 to 4 C atoms,

characterised in that optically active amino acid derivatives of the formula (II)

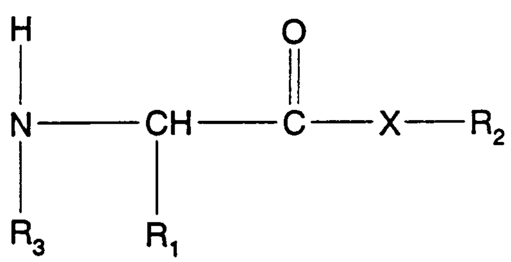

(II)

in which $R_1$, $R_2$ and $R_3$ are as defined under formula (I),

or their acid addition products are reacted with fluoroacryloyl derivatives of the formula

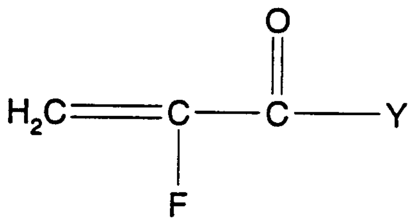

(III)

in which Y represents fluorine, chlorine or bromine,
if appropriate in the presence of an acid-binding agent in inert organic solvents.

**2.** Process for the preparation of compounds according to Claim 1, characterised in that compounds of the formula (II) are reacted with a fluorine compound of the formula (IV)

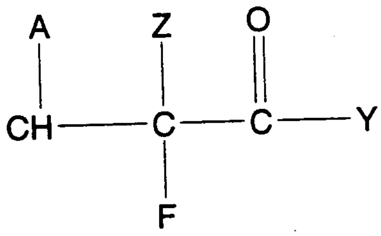

(IV)

in which
Y is as defined above, and

A and Z each represent hydrogen, fluorine, chlorine or bromine, in which A and Z never simultaneously denote hydrogen,
with elimination of AZ to give fluoroacrylic compounds of the formula (III), if appropriate in the presence of inert organic solvents.

**3.** Process for the preparation of optically active polymers containing at least 40 mol% of structural units of the formula (V)

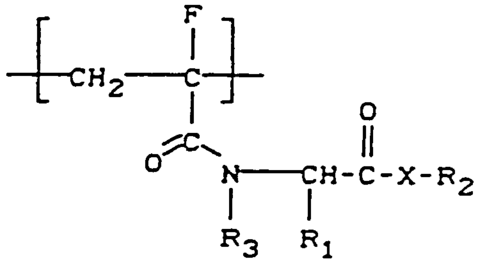

(V)

in which R, $R_1$, $R_2$, $R_3$ and X are as defined in Claim 1, characterised in that the N-α-fluoroacryloylamino acid derivatives of the general formula (I) are prepared in the presence of 0.5 to 50 mol% of a crosslinking agent by suspension polymerisation, if appropriate in the presence of free-radical formers and inert organic solvents.

**Revendications**

**Revendications pour les Etats contractants suivants : AT, BE, CH, DE, DK, FR, GB, IT, LI, LU, NL, SE**

**1.** Dérives de N-α-fluoracryloylaminoacides optiquement actifs, repondant à la formule (I)

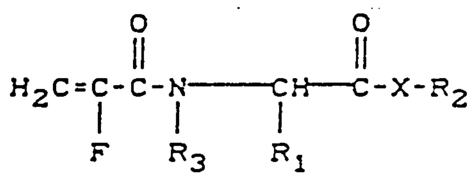

(I),

dans laquelle

- $R_1$ représente un radical alkyle en $C_1$-$C_8$ à chaîne droite ou ramifiée, un radical aralkyle en $C_7$-$C_{12}$, un radical cycloalkyle en $C_3$-$C_{10}$, un radical aryle en $C_6$-$C_{14}$ ou encore un radical furyle, un radical thiényle ou un radical pyridyle, qui sont éventuellement substitués par un groupe benzyloxycarbonyle, par un groupe alcoxycarbonyle contenant jusgu'a 6 atomes de carbone, par un groupe hydroxyle, par un groupe alkyle, par un groupe cycloalkyle ou par un groupe alcoxy contenant respectivement jusqu'a 6 atomes de carbone, par un atome d'halogène, par un groupe phénoxy, par un groupe benzoxy, par un groupe acylamino contenant jusqu'a 8 atomes de carbone ou encore par un groupe carbonylalcoxy contenant jusqu'à 6 atomes de carbone,
- $R_3$ représente un atome d'hydrogène ou un groupe alkyle en $C_1$-$C_4$, ou bien forme, ensemble avec $R_1$, un groupe tri- ou tétraméthylène,
- X représente un atome d'oxygène ou un groupe $NR_4$ dans lequel
- $R_4$ représente un atome d'hydrogène ou un groupe alkyle en $C_1$-$C_4$ à chaîne droite ou ramifiée ou bien forme, ensemble avec $R_2$ et l'atome d'azote, un noyau penta- à heptagonal qui est éventuellement substitué par un groupe alcoxycarbonyle contenant jusqu'à 6 atomes de carbone ou encore par un ou deux groupes alkyle contenant chacun de 1 à 4 atomes de carbone,
- $R_2$ représente un radical alkyle en $C_1$-$C_{22}$ à chaîne droite ou ramifiée, un radical aralkyle en $C_7$-$C_{12}$, un radical cycloalkyle en $C_3$-$C_{10}$, un radical aryle en $C_6$-$C_{14}$ ou encore un radical terpényle, qui sont éventuellement substitués par un atome d'halogène, par un groupe alkyle ou par un groupe alcoxy contenant respectivement de 1 à 4 atomes de carbone.

**2.** Composés répondant à la formule générale (I) selon la revendication 1, dans lesquels

- $R_1$ représente un groupe alkyle contenant de 1 à 4 atomes de carbone, un groupe benzyle, un groupe cycloalkyle contenant de 3 à 7 atomes de carbone, un groupe phényle, un groupe furyle, un groupe indolylméthyle, un groupe naphtyle, un groupe naphtylméthyle, un groupe thiényle ou un groupe pyridyle, dans lesquels les radicaux alkyle et aryle mentionnés sont éventuellement substitués une ou deux fois par un groupe hydroxyle, par un groupe méthoxy, par un atome d'halogène, par un groupe alkyle, par un groupe cycloalkyle contenant jusqu'à 6 atomes de carbone, par un groupe phénoxy, par un groupe benzoxy, par un groupe acétylamino, par un groupe benzyloxycarbonyle ou par un groupe alcoxycarbonyle contenant jusqu'à 4 atomes de carbone,
- $R_3$ représente un atome d'hydrogène, un groupe méthyle ou un groupe éthyle ou bien forme, ensemble avec $R_1$, un groupe tri- ou tétraméthylène,
- X représente un atome d'oxygène ou un groupe $NR_4$ dans lequel
- $R_4$ représente un atome d'hydrogène ou un groupe alkyle contenant de 1 à 4 atomes de carbone ou bien forme, ensemble avec $R_2$ et l'atome d'azote, un noyau penta- à heptagonal qui est éventuellement substitué par un groupe alkyle ou par un groupe alcoxycarbonyle contenant respectivement jusqu'à 4 atomes de carbone, et
- $R_2$ représente un groupe alkyle contenant jusqu'à 12 atomes de carbone, un groupe benzyle, un groupe cycloalkyle contenant de 3 à 7 atomes de carbone, un groupe phényle ou un groupe terpényle, dans lesquels les radicaux mentionnés sont éventuellement substitués par un atome de fluor, par un atome de chlore ou par un atome de brome ou encore par un groupe

alkyle ou par un groupe alcoxy contenant respectivement jusqu'à 2 atomes de carbone.

**3.** Composés répondant à la formule générale (I) selon la revendication 1, caractérisés en ce qu'ils contiennent la séquence d'aminoacides des aminoacides optiquement actifs alanine, acide aminobutyrique, valine, norvaline, leucine, isoleucine, terleucine, phénylglycine, naphtylglycine, phénylalanine, thiénylalanine, pyridylalanine, naphtylalanine, cyclohexylglycine, cyclohexylalanine, tyrosine, tryptophane, sérine, acide asparagique, acide glutamique, ornithine, lysine, proline ou encore l'acide 6-aminopénicillanique.

**4.** Procédé pour la préparation de composés répondant à la formule générale (I) selon la revendication 1, caractérisé en ce que
A) on fait réagir des dérivés d'aminoacides optiquement actifs répondant à la formule (II)

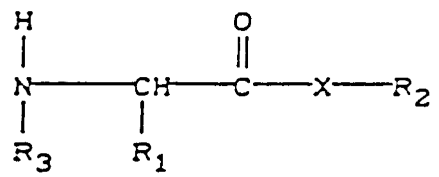

(II)

dans laquelle
$R_1$, $R_2$ et $R_3$ ont la signification indiquée en dessous de la formule (I)
ou leurs produits d'addition d'acides,
avec des dérivés fluoracryloyliques répondant à la formule

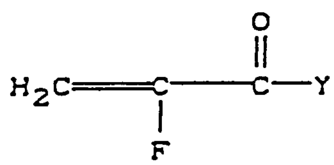

(III)

dans laquelle
Y représente un atome de fluor, un atome de chlore ou un atome de brome,
éventuellement en présence d'un agent neutralisateur d'acides, dans des solvants organiques inertes,
ou
B) on fait réagir des composés de formule (II) avec un composé fluoré de formule (IV)

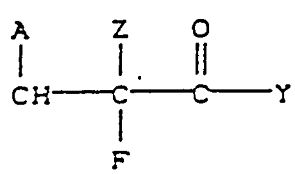

(IV)

dans laquelle
Y a la signification indiquée ci-dessus, et
A et Z représentent respectivement un atome d'hydrogène, un atome de fluor, un atome de chlore ou un atome de brome, A et Z ne pouvant jamais représenter simultanément un atome d'hydrogène,
avec séparation de AZ pour obtenir des composés fluoracryliques de formule (III), éventuellement en présence de solvants organiques inertes.

**5.** Polymères optiquement actifs contenant au moins 40 moles % d'unités de structure de formule (V)

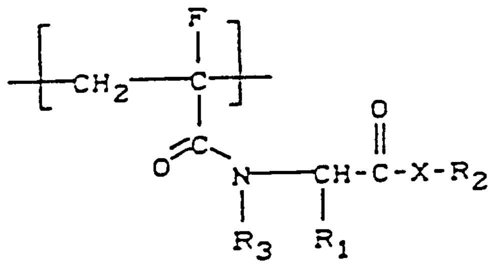

(V)

dans laquelle
R, $R_1$, $R_2$, $R_3$ et X ont la signification indiquée à la revendication 1.

**6.** Polymères optiquement actifs selon la revendication 5, caractérisés en ce qu'ils sont présents sous la forme de polymères réticulés en perles, insolubles ou gonflables, ou encore sous une forme liée à des matières de support inorganiques à grains fins.

**7.** Procédé pour la préparation de polymères réticulés en perles selon la revendication 6, caractérisé en ce qu'on prépare les dérivés de N-$\alpha$-fluoracryloylaminoacides répondant à la formule générale (I) avec de 0,5 à 50 moles % d'un agent de réticulation par polymérisation en suspension, éventuellement en présence de formateurs de radicaux et de solvants organiques inertes.

**8.** Procédé pour la préparation de polymères optiquement actifs liés à des matières de support inorganiques selon la revendication 6, caractérisé en ce qu'on estérifie des composés répondant à la formule générale (I) selon la revendication 1 en présence de gels de silice vinyliques ou de phases de gel de silice-diol portant éventuellement un substituant (méth)acryloyle, éventuellement en présence de formateurs de radicaux et éventuellement en présence de solvants organiques inertes.

**9.** Utilisation de polymères selon la revendication 7, pour la séparation d'isomères optiques.

**Revendications pour les Etats contractants suivants : ES, GR**

**1.** Procédé pour la préparation de dérivés de N-$\alpha$-fluoracryloylaminoacides optiquement actifs, répondant à la formule (I)

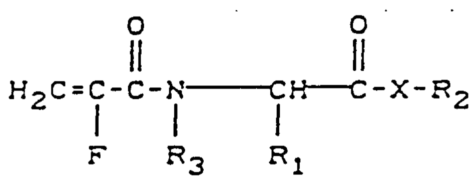

(I),

dans laquelle

$R_1$ représente un radical alkyle en $C_1$-$C_8$ à chaîne droite ou ramifiée, un radical aralkyle en $C_7$-$C_{12}$, un radical cycloalkyle en $C_3$-$C_{10}$, un radical aryle en $C_6$-$C_{14}$ ou encore un radical furyle, un radical thiényle ou un radical pyridyle, qui sont éventuellement substitués par un groupe benzyloxycarbonyle, par un groupe alcoxycarbonyle contenant jusqu'à 6 atomes de carbone, par un groupe hydroxyle, par un groupe alkyle, par un groupe cycloalkyle ou par un groupe alcoxy contenant respectivement jusqu'à 6 atomes de carbone, par un atome d'halogène, par un groupe phénoxy, par un groupe benzoxy, par un groupe acylamino contenant jusqu'à 8 atomes de carbone ou encore par un groupe carbonylalcoxy contenant jusqu'à 6 atomes de carbone,

$R_3$ représente un atome d'hydrogène ou un groupe alkyle en $C_1$-$C_4$, ou bien forme, ensemble avec $R_1$, un groupe tri- ou tétraméthylène,

X représente un atome d'oxygène ou un groupe $NR_4$ dans lequel

$R_4$ représente un atome d'hydrogène ou un groupe alkyle en $C_1$-$C_4$ à chaîne droite ou ramifiée ou bien forme, ensemble avec $R_2$ et l'atome d'azote, un noyau penta- à heptagonal qui est éventuellement substitué par un groupe alcoxycarbonyle contenant jusqu'à 6 atomes de carbone ou encore par un ou deux groupes alkyle contenant chacun de 1 à 4 atomes de carbone,

$R_2$ représente un radical alkyle en $C_1$-$C_{22}$ à chaîne droite ou ramifiée, un radical aralkyle en $C_7$-$C_{12}$, un radical cycloalkyle en $C_3$-$C_{10}$, un radical aryle en $C_6$-$C_{14}$ ou encore un radical terpényle, qui sont éventuellement substitués par un atome d'halogène, par un groupe alkyle ou par un groupe alcoxy contenant respectivement de 1 à 4 atomes de carbone,

caractérisé en ce qu'on fait réagir des dérivés d'aminoacides optiquement actifs répondant à la formule (II)

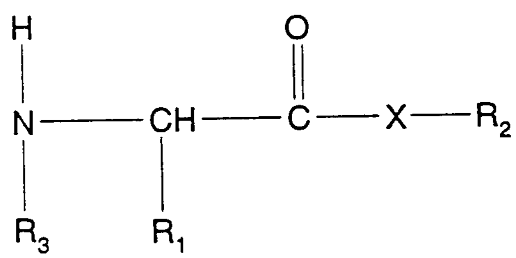

(II)

dans laquelle
$R_1$, $R_2$ et $R_3$ ont la signification indiquée en dessous de la formule (I)
ou leurs produits d'addition d'acides, avec des dérivés fluoracryloyliques répondant à la formule

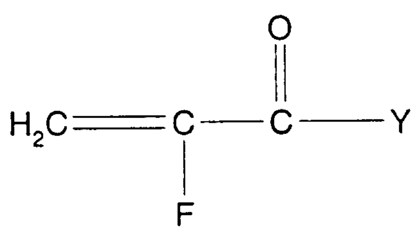

(III)

dans laquelle Y représente un atome de fluor, un atome de chlore ou un atome de brome, éventuellement en présence d'un agent neutralisateur d'acides, dans des solvants organiques inertes.

**2.** Procédé pour la préparation de composés selon la revendication 1, caractérisé en ce qu'on fait réagir des composés de formule (II) avec un composé fluoré de formule (IV)

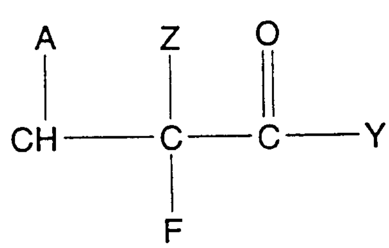

(IV)

dans laquelle

Y a la signification indiquée ci-dessus, et

A et Z représentent respectivement un atome d'hydrogène, un atome de fluor, un atome de chlore ou un atome de brome, A et Z ne pouvant jamais représenter simultanément un

atome d'hydrogène,
avec séparation de AZ pour obtenir des composés fluoracryliques de formule (III), éventuellement en présence de solvants organiques inertes.

3. Procédé pour la préparation de polymères optiquement actifs contenant au moins 40 moles % d'unités de structure de formule (V)

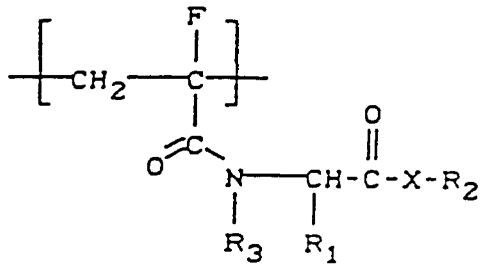

(V)

dans laquelle
R, $R_1$, $R_2$, $R_3$ et X ont la signification indiquée à la revendication 1, caractérisé en ce qu'on prépare les dérivés de N-α-fluoracryloylaminoacides répondant à la formule générale (I) avec de 0,5 à 50 moles % d'un agent de réticulation par polymérisation en suspension, éventuellement en présence de formateurs de radicaux et de solvants organiques inertes.